(19) Europäisches Patentamt / European Patent Office / Office européen des brevets

(11) **EP 4 062 172 B1**

(12) **FASCICULE DE BREVET EUROPEEN**

(45) Date de publication et mention de la délivrance du brevet:
**03.01.2024 Bulletin 2024/01**

(21) Numéro de dépôt: **20808113.3**

(22) Date de dépôt: **18.11.2020**

(51) Classification Internationale des Brevets (IPC):
**G01N 33/00** $^{(2006.01)}$

(52) Classification Coopérative des Brevets (CPC):
**G01N 33/0075; G01N 33/0034**

(86) Numéro de dépôt international:
**PCT/EP2020/082599**

(87) Numéro de publication internationale:
**WO 2021/099427 (27.05.2021 Gazette 2021/21)**

(54) **PROCÉDÉ D'ESTIMATION DE LA CONCENTRATION D'ANALYTES DANS L'AIR AU VOISINAGE D'UNE VOIE PARCOURUE PAR DES MOYENS DE TRANSPORT**

VERFAHREN ZUR ABSCHÄTZUNG DER KONZENTRATION VON ANALYTEN IN LUFT IN DER NÄHE EINES VON EINEM VERKEHRSMITTEL BEFAHRENEN FAHRWEGS

METHOD FOR ESTIMATING THE CONCENTRATION OF ANALYTES IN AIR CLOSE TO A ROAD USED BY MEANS OF TRANSPORT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **22.11.2019 FR 1913131**

(43) Date de publication de la demande:
**28.09.2022 Bulletin 2022/39**

(73) Titulaire: **Elichens**
**38040 Grenoble (FR)**

(72) Inventeur: **ZANINI, Paolo**
**38100 GRENOBLE (FR)**

(74) Mandataire: **INNOV-GROUP**
**310, avenue Berthelot**
**69372 Lyon Cedex 08 (FR)**

(56) Documents cités:
**CN-B- 106 056 210**

• **A. SULEIMAN ET AL: "Applying machine learning methods in managing urban concentrations of traffic-related particulate matter (PM10 and PM2.5)", ATMOSPHERIC POLLUTION RESEARCH, vol. 10, no. 1, 6 juillet 2018 (2018-07-06), pages 134-144, XP055730747, ISSN: 1309-1042, DOI: 10.1016/j.apr.2018.07.001**

**Description**

## DOMAINE TECHNIQUE

Le domaine technique de l'invention concerne une estimation de la concentration d'analytes dans l'air au niveau ou au voisinage d'une rue.

## ART ANTERIEUR

Les préoccupations concernant la qualité de l'air sont de plus en plus importantes. Afin de répondre à une attente de la population, ou des autorités, on a développé des moyens d'établir des cartographies décrivant la distribution spatiale de concentrations en molécules ou en particules nocives dans l'air, en particulier dans les villes.

De nombreux modèles ont été développés, permettant d'établir des cartographies de pollution atmosphérique et de prévoir leurs évolutions temporelles. Il est alors possible de modéliser la pollution de l'air en milieu urbain en situation de routine, ou en situation accidentelle, par exemple suite à un accident chimique ou nucléaire. La couverture géographique peut se limiter à quelques $km^2$, voire à l'échelle d'un pays ou d'un continent dans les applications visant à modéliser le transport à grande échelle de polluants.

Certains modèles de prédiction sont essentiellement basés sur la dynamique des fluides. Il s'agit par exemple du modèle paramétrique OSPM (Danish Operational Street Pollution Model). Un tel modèle est alimenté par des données relatives au trafic routier. Sur la base de la modélisation de courants de recirculation, un tel modèle permet de prévoir, de façon satisfaisante, une dispersion de polluants dans un certain type de rues, désignées par le terme "street canyon". Lorsqu'on s'écarte de la configuration géométrique correspondant à la configuration "street canyon", la validité du modèle peut être affectée. C'est notamment le cas lorsque la rue considérée est une large avenue.

Des agences régionales ou nationales exploitent des stations de mesure réparties sur le territoire, dites stations de référence, qui permettent d'obtenir des mesures régulières de la concentration de polluants atmosphériques. Ces derniers sont par exemple $NO_2$, $O_3$, CO, ou encore des particules fines, par exemple des particules de diamètre inférieur ou égal à 10 $\mu$m (PM 10) ou des particules de diamètre inférieur à 2.5 $\mu$m (PM 2.5). Les mesures réalisées par certaines agences sont ouvertes, c'est-à-dire aisément accessibles au public. Au niveau européen, il est par exemple possible d'obtenir les concentrations de ces polluants sur le site internet de l'agence européenne de l'environnement. En France, des agences régionales gèrent des stations de mesure, ce qui permet d'obtenir des cartographies de polluants ainsi que des prévisions. Les stations de mesures sont des dispositifs fiables, mais onéreux et encombrants. De ce fait, il est difficile d'envisager leur déploiement selon un maillage spatial fin. Leur nombre se limite à quelques unités par agglomération, voire 10 à 20 pour les agglomérations les plus importantes.

D'autres approches sont décrites dans CN 106 056 210 et dans A. SULEIMAN ET AL: "Applying machine learning methods in managing urban concentrations of traffic-related particulate matter (PM10 and PM2.5)", ATMOSPHERIC POLLUTION RESEARCH, vol. 10, no. 1, 6 juillet 2018 (2018-07-06), pages 134-144, XP055730747,ISSN: 1309-1042, DOI: 10.1016/j.apr.2018.07.001.

En se basant sur des mesures de stations de référence, et en utilisant des données relatives au trafic routier, il est possible d'obtenir une cartographie. Cependant, la résolution spatiale, ainsi que la précision d'estimation, dépend de la densité d'implantation des stations de référence. Or, cette densité est généralement trop faible pour obtenir une cartographie précise de haute résolution.

Les inventeurs ont proposé un procédé permettant d'obtenir une cartographie, de la concentration d'au moins un analyte, et de préférence de plusieurs analytes, dans un milieu urbain. La résolution spatiale de la cartographie peut être élevée. La cartographie obtenue peut être renouvelée à intervalles de temps réguliers plusieurs fois par jour. Elle tient compte des conditions locales rencontrées au niveau de chaque point d'estimation.

## EXPOSE DE L'INVENTION

Un premier objet de l'invention est un procédé d'estimation d'une concentration d'au moins deux analytes dans un environnement, notamment un environnement urbain ou péri-urbain, comportant des voies configurées pour être traversées par un trafic de véhicules, le procédé comportant les étapes suivantes :

a) maillage spatial de l'environnement, de façon à obtenir des points de maillage ;
b) prise en compte, au niveau de chaque point de maillage, à un instant d'estimation :

- de préférence, d'au moins une donnée fixe dans le temps, relative à chaque voie ;
- de données dynamiques, variables en fonction de l'instant d'estimation, comportant des données météorologiques, des données relatives au trafic, et des données temporelles relatives à l'instant d'estimation ;

c) utilisation des données prises en compte lors de l'étape b), à l'instant d'estimation, en tant que données d'entrée d'un premier algorithme d'intelligence artificielle supervisé, de façon à estimer, au point de maillage, ou à chaque point du maillage, une concentration d'au moins un premier analyte, à l'instant d'estimation;

d) utilisation de la concentration d'un premier analyte, à au moins un point de maillage, à l'instant d'estimation, en tant que donnée d'entrée d'un deuxième algorithme d'intelligence artificielle supervisé, différent du premier algorithme d'intelligence artificielle supervisé, de façon à estimer, au point de maillage, une concentration d'au moins un deuxième analyte, différent du premier analyte, à l'instant d'estimation.

Les voies peuvent notamment être des routes. Le trafic peut être un trafic routier. L'environnement urbain ou péri-urbain peut être une ville, une zone commerciale disposée à proximité d'une ville, une zone aéroportuaire, ou un environnement naturel traversé par une voie de circulation importante, par exemple une autoroute.

L'analyte peut être une molécule de gaz ou une particule de matière, considérée comme polluante.

De préférence, la concentration du deuxième analyte est considérée comme corrélée avec la concentration du premier analyte.

Le procédé peut comporter l'une quelconque des caractéristiques suivantes, prises isolément ou selon les combinaisons possibles :

- Lors de l'étape b), les données météorologiques comportent :

  • la direction du vent ;
  • et/ou la vitesse du vent.

- Les données relatives au trafic routier comportent au moins une vitesse moyenne des véhicules et/ou un niveau d'intensité du trafic.
- Les données météorologiques comportent également au moins une des données suivantes : température ; pression ; taux d'humidité ; nébulosité.
- Les données temporelles correspondant à l'instant d'estimation comportent au moins une des données suivantes :

  • type de journée parmi des types de journées prédéterminés à laquelle appartient l'instant d'estimation ;
  • segment temporel auquel appartient l'instant d'estimation, chaque journée étant divisée en segments temporels.

- Les types de journées prédéterminés peuvent comporter : jour compris entre le lundi et le vendredi ; samedi ; dimanche.
- Lorsque la journée est divisée en segments temporels, chaque segment temporel peut avoir une durée d'une heure.
- Le premier algorithme est un réseau de neurones, comportant :

  • une couche d'entrée, comportant les données d'entrée prises en compte lors de l'étape b) ;
  • au moins une couche cachée, et de préférence au moins deux couches cachées, comportant des noeuds ;
  • une couche de sortie, comportant une estimation d'une concentration du premier analyte ou de chaque premier analyte.

- Le premier réseau de neurones comporte deux couches cachées.
- La première couche cachée du premier réseau de neurones comporte entre 200 et 800 noeuds.
- la deuxième couche cachée du premier réseau de neurones comporte entre 2500 et 4500 noeuds.
- Le deuxième algorithme est un deuxième réseau de neurones comportant :

  • une couche d'entrée, comportant des données d'entrée prises en compte lors de l'étape b), ainsi qu'une concentration d'un premier analyte, estimée à l'instant d'estimation, au cours de l'étape c);
  • au moins une couche cachée, et de préférence au moins deux couches cachées, comportant des noeuds ;
  • une couche de sortie, comportant une estimation d'une concentration du deuxième analyte, à l'instant d'estimation.

- Le deuxième réseau de neurones comporte deux couches cachées.
- La première couche cachée du deuxième réseau de neurones comporte entre 200 et 800 noeuds.
- la deuxième couche cachée du deuxième réseau de neurones comporte entre 2500 et 4500 noeuds.
- L'étape d) comporte une estimation de la concentration d'au moins deux deuxièmes analytes différents, en utilisant respectivement au moins deux deuxièmes réseaux de neurones différents, chaque deuxième réseau de neurones permettant une estimation d'une concentration d'un deuxième analyte.

L'invention sera mieux comprise à la lecture de l'exposé des exemples de réalisation présentés, dans la suite de la description, en lien avec les figures listées ci-dessous.

## FIGURES

La figure 1 montre un exemple de points de maillage dans un environnement urbain.
La figure 2 schématise une architecture d'un réseau de neurones.
La figure 3 représente les principales étapes d'un procédé selon l'invention.
La figure 4A montre un exemple de comparaisons entre des estimations d'une concentration de $NO_2$ à l'aide d'un procédé selon l'invention, avec des mesures de référence et des estimations effectuées à l'aide d'un modèle de l'art antérieur.
La figure 4B montre un exemple de comparaisons entre des estimations d'une concentration de PM10 à l'aide d'un procédé selon l'invention, avec des mesures de référence et des estimations effectuées à l'aide d'un modèle de l'art antérieur.

## EXPOSE DE MODES DE REALISATION PARTICULIERS

L'invention s'applique en particulier à un environnement urbain ou périurbain, comportant des rues au niveau desquelles on cherche à estimer une concentration, en au moins deux analytes, dans l'air. L'analyte peut être une espèce gazeuse, par exemple CO, NO, $NO_z$, $O_3$, $SO_z$, $C_6H_6$ ou des particules fines, par exemple des particules de type PM10 ou PM2.5. L'acronyme PM, connu de l'homme du métier, signifie Particulate Matter, ou matière particulaire (ou particule fine). Les nombres 10 ou 2.5 correspondent au diamètre, exprimé en $\mu m$.

D'une manière générale, l'environnement considéré comporte des sources d'émission de l'analyte. Ces sources d'émission peuvent être notamment liées à un trafic de véhicules, en particulier des véhicules terrestres ou aériens susceptibles d'émettre l'analyte. L'environnement peut également être affecté par la présence d'installations de chauffage urbain, ou à la présence d'installations industrielles susceptibles d'émettre l'analyte.

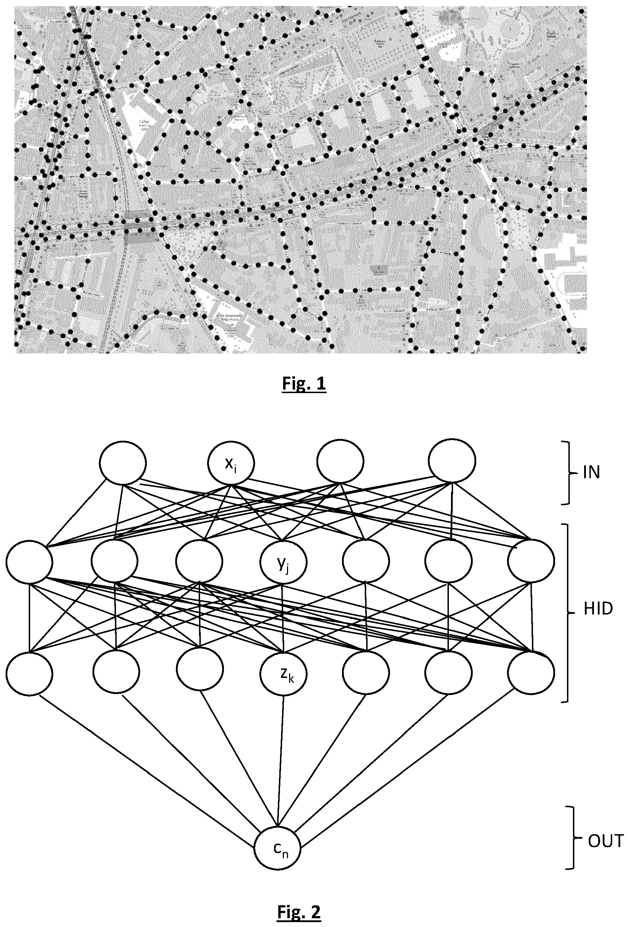

L'environnement étudié fait l'objet d'un maillage spatial. Il s'agit de diviser l'environnement en différentes mailles, chaque maille correspondant à un point de maillage au niveau duquel on cherche à estimer une concentration de l'analyte. La figure 1 représente un tel maillage. La résolution spatiale du maillage est de préférence telle que selon au moins une direction, la longueur d'une maille est inférieure ou égale à 500 m, voire inférieure ou égale à 250 m. Les inventeurs ont développé un procédé permettant d'estimer, en au moins une maille, et de préférence en chaque maille, une concentration d'au moins deux analytes, et de préférence de plusieurs analytes, dans l'air.

Plutôt que d'établir un modèle analytique, les inventeurs ont choisi le recours à un algorithme d'intelligence artificielle supervisé pour estimer la concentration de chaque analyte. Dans les exemples qui suivent, l'algorithme d'intelligence artificielle supervisé est un réseau de neurones.

L'algorithme est mis en oeuvre par une unité de traitement, comportant un processeur, par exemple un microprocesseur. L'unité de traitement est configurée pour mettre en oeuvre des instructions, stockées dans une mémoire, pour effectuer le procédé décrit par la suite. Pour cela, l'unité de traitement reçoit des données d'entrée, qui correspondent à des grandeurs physiques mesurées ou observées. Les données d'entrée sont notamment issues de bases de données publiques ainsi que d'une horloge, cette dernière permettant de définir la journée et l'horaire auquel l'estimation est effectuée.

La figure 2 illustre une architecture d'un réseau de neurones. Ce type d'architecture, connue de l'homme du métier, comporte une couche d'entrée IN, comportant des données d'entrée, au moins une couche intermédiaire HID, ou couche cachée, et une couche de sortie OUT, comportant la grandeur à estimer, en l'occurrence la concentration d'un analyte, ou de plusieurs analytes. Les inventeurs ont estimé que pour l'application visée, le nombre de couches intermédiaires (ou couches cachées) pouvait être égal à 2.

L'algorithme est de préférence mis en oeuvre en différents points du maillage, et en différents instants d'estimation. A chaque instant d'estimation, l'algorithme est mis en oeuvre indépendamment pour chaque point du maillage considéré.

Les données d'entrées, formant la couche d'entrée IN, comprennent des grandeurs physiques mesurées ou estimées. Les inventeurs ont considéré qu'il était optimal de considérer 5 types de données :

- des données d'entrée relatives à l'orientation de la rue ou à sa géométrie : il s'agit de données d'entrées fixes, propres à chaque rue, considérées comme invariables dans le temps ;
- des données d'entrée relatives au trafic ;
- des données d'entrée météorologiques ;

- des données d'entrée relative à une pollution atmosphérique de fond, affectant l'environnement considéré dans son ensemble ;
- des données d'entrée relatives à l'instant auquel l'estimation est effectuée : il s'agit en particulier du type de journée (jour de travail, jour de repos) et de l'horaire.

Les données d'entrée relatives à la pollution atmosphérique de fond peuvent être issues d'un modèle à large échelle, par exemple le modèle Copernicus, développé par "The Copernicus Atmopshere Monitoring Service", et dont les données sont aisément accessibles. Les données fournies par ce modèle peuvent faire l'objet d'un recalage local, en considérant des stations de mesure de référence localisées à proximité de l'environnement considéré.

Les données d'entrée sont établies en chaque point du maillage considéré, et à chaque instant d'estimation. Les données d'entrée relatives au trafic, météorologiques, relatives à la pollution atmosphérique de fond, ou à l'instant auquel l'estimation est effectuée, sont dynamiques : elles peuvent varier en fonction de l'instant d'estimation.

La couche d'entrée IN peut comporter d'autres données d'entrée, par exemple des mesures d'autres analytes, que l'on considère comme corrélés à l'analyte que l'on cherche à quantifier. La couche d'entrée peut également comporter des mesures de l'analyte effectuée par des capteurs considérés comme proches du point de mesure.

Chaque couche cachée HID forme comporte des noeuds ou neurones. Le nombre de noeuds par couche peut être déterminé de façon expérimentale par l'homme du métier, ou au cours d'une étape d'apprentissage. Comme précédemment indiqué, pour cette application, les inventeurs ont estimé qu'il était optimal que le nombre de couches cachées soit égal à 2. Chaque noeud est alimenté par des noeuds provenant d'une couche de rang inférieur. Dans l'exemple représenté, la première couche cachée comporte $N_j$ noeuds $y_j$. La deuxième couche cachée comporte $N_k$ noeuds $z_k$. La couche d'entrée comporte $N_i$ données d'entrée $x_i$. La couche de sortie comporte $N_n$ données de sortie $c_n$.

De façon connue par l'homme du métier, on détermine une valeur de chaque noeud en appliquant une fonction d'activation à une combinaison linéaire des valeurs des noeuds d'une couche précédente.

Ainsi, pour la première couche cachée, la valeur de chaque noeud $y_j$ est telle que :

$$y_j = f_j \left( \sum_i (w_{j,i} x_i + b_i) \right) \quad (1)$$

où

- $x_i$ est la valeur de chaque donnée d'entrée considérée ;
- $b_i$ est un biais associé à chaque donnée d'entrée $x_i$;
- $f_j$ est une fonction d'activation associé au noeud de rang $j$ de la première couche cachée;
- $w_{j,i}$ est un terme de pondération pour la donnée d'entrée de rang $i$ et le noeud de rang $j$ de la première couche cachée.

De même, pour la deuxième couche cachée, la valeur de chaque noeud $z_k$ est telle que :

$$z_k = f_k \left( \sum_j (w_{k,j} y_j + b_j) \right) \quad (2)$$

où

- $y_j$ est la valeur de chaque noeud de la première couche cachée;
- $b_j$ est un biais associé à chaque noeud de la première couche cachée ;
- $f_k$ est une fonction d'activation associé au noeud de rang k de la deuxième couche cachée ;
- $w_{k,j}$ est un terme de pondération pour le noeud de rang $j$ de la première couche cachée et le noeud de rang $k$ de la deuxième couche cachée.

Enfin, la valeur de chaque noeud de la couche de sortie $c_n$ est tel que

$$c_n = f_n \left( \sum_k (w_{n,k} z_k + b_k) \right) \quad (3)$$

où

- $z_k$ est la valeur de chaque noeud de la deuxième couche cachée;
- $b_k$ est un biais associé à chaque noeud de la deuxième couche cachée ;
- $f_n$ est une fonction d'activation associé à la donnée de sortie de rang n ;
- $w_{n,k}$ est un terme de pondération pour le noeud de rang $k$ de la deuxième couche cachée et le noeud de rang $n$ de la couche de sortie.

La couche de sortie comporte à une estimation d'une concentration $c_n$ d'un analyte $n$. La couche de sortie peut ne comporter qu'un seul terme, ou plusieurs termes, chaque terme correspondant à une estimation d'une concentration. Dans l'exemple représenté sur la figure 2, la couche de sortie ne comporte qu'un seul terme.

La forme des fonctions d'activation est prédéfinie par l'homme du métier. Dans cet exemple, on a considéré que chaque fonction d'activation $f_p$ au niveau d'un noeud d'une couche de rang $p$ est telle que :

$$f_p = \max\left(0, \sum_q (w_{p,q} a_q + b_q)\right) \ (4)$$

où $a_q$ est une valeur du noeud de rang q de la couche précédente et $b_q$ est le biais correspondant.

L'architecture du réseau de neurones peut être paramétrée par un algorithme dédié sous l'environnement MATLAB ou l'environnement PYTHON.

Chaque fonction d'activation est déterminée au cours d'une phase d'apprentissage. Au cours d'une telle phase, les données de sorties ainsi que les données d'entrée sont connues. Les inventeurs ont effectué une phase d'apprentissage en utilisant des concentrations d'analyte établies par des stations de mesure de référence, respectivement considérées comme représentatives de différentes rues. Il s'agit de stations de mesure de type "trafic", c'est-à-dire identifiées comme étant impactées par le trafic. Cela signifie que les mesures fournies par ces stations de référence fluctuent en fonction du trafic. Les concentrations d'analyte ont été mesurées en différents instants d'apprentissage. A chaque instant d'apprentissage, on a établi des données d'entrée, comportant notamment les données d'entrée préalablement discutées. La phase d'apprentissage permet :

- d'établir les valeurs des biais et des facteurs de pondération des expressions (1), (2) et (3) ;
- de définir des valeurs optimales du nombre de noeuds comportant chaque couche cachée ;
- d'identifier les données d'entrée les plus pertinentes.

Dans un premier mode de réalisation, on a considéré une donnée de sortie unique, correspondant à une estimation de la concentration d'un analyte. Selon ce mode de réalisation, chaque analyte est associé à un réseau de neurones. Dans un deuxième mode de réalisation, on a considéré une donnée de sortie comportant différents termes, chaque terme correspondant à une estimation de la concentration d'un analyte. Selon ce mode de réalisation, un même réseau de neurones permet d'adresser simultanément plusieurs analytes.

Durant la phase d'apprentissage, les analytes adressés étaient $NO_2$ et PM10 (Particules de matière de diamètre inférieur à 10 $\mu$m).

Certains analytes sont corrélés à d'autres analytes. C'est par exemple le cas de $O_3$, qui dépend de $NO_2$ et des conditions d'ensoleillement. En effet,

$NO_2 + hv + O_2 \rightarrow O_3 + NO$, où hv correspond à l'énergie lumineuse.

C'est également le cas de PM2.5, dont la concentration est corrélée avec la concentration de PM10.

Aussi, le procédé développé par les inventeurs peut comporter une sélection de premiers analytes et de deuxièmes analytes, les deuxièmes analytes étant considérés comme corrélés avec les premiers analytes. A l'aide d'un premier réseau de neurones, tel que schématisé sur la figure 2, on estime une concentration d'au moins un premier analyte. A l'aide d'un deuxième réseau de neurones, on estime une concentration d'au moins un deuxième analyte. Le deuxième réseau de neurones peut avoir une structure similaire au premier réseau de neurones, c'est-à-dire une couche d'entrée, une couche de sortie comportant l'estimation de la concentration du deuxième analyte, ainsi que deux couches cachées entre la couche d'entrée et la couche de sortie. Les données d'entrée du deuxième réseau de neurones comportent au moins une estimation de la concentration d'un premier analyte, estimée par le premier réseau de neurones.

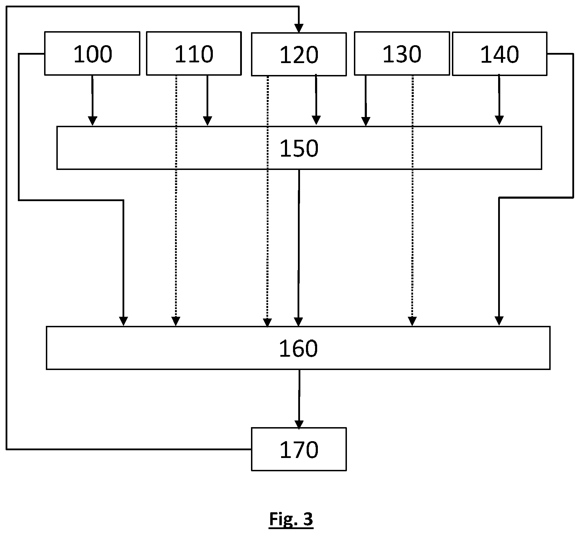

Les principales étapes du procédé sont représentées sur la figure 3, et sont à présent décrites. Ces étapes sont mises en oeuvre au niveau de chaque point issu d'un maillage préalable de l'environnement. Elles sont mises en oeuvre à chaque instant d'estimation.

Etape 100 : prise en compte de données d'entrée fixes, relatives à la rue considérée

Ces données peuvent comporter une orientation de la rue, cette valeur étant utilisée déterminer un angle par rapport à une direction du vent. Ces données peuvent également comprendre une variable catégorielle, dont la valeur dépend d'une catégorie attribuée à la rue. Par exemple, lorsqu'on utilise la base de données HERE (Here technologies), on peut utiliser une variable catégorielle usuellement désignée par l'acronyme "FC" (Functional Class), quantifiant la classe de la rue. Chaque rue a préalablement fait l'objet d'une classification. L'indice FC peut par exemple varier entre 1 et 5, selon que la rue soit une autoroute (FC = 1) ou une route à circulation réduite (FC = 5).

Etape 110 : prise en compte de données météorologiques

Les données météorologiques prises en compte sont le niveau d'humidité, la température, la pression atmosphérique, la vitesse du vent, l'intensité des précipitations, la nébulosité, un angle entre la direction du vent et l'orientation de la rue. L'intensité des précipitations correspond par exemple à une quantité horaire de précipitation. La nébulosité quantifie la couverture nuageuse. Elle peut être prendre la forme d'un indice compris entre 0 (absence de nuage) à 8 (couverture considérée comme maximale). L'unité peut être l'octa, qui est une unité usuellement utilisée en météorologie. Ces données sont disponibles auprès de stations de mesure météorologiques disposées dans l'environnement examiné. Elles sont généralement communes pour différentes mailles, voire pour l'ensemble des mailles considérées. Lorsque l'environnement modélisé comporte différentes stations météorologiques, les données météorologiques peuvent faire l'objet d'une interpolation dans le maillage considéré. Par exemple une interpolation linéaire.

Etape 120 : prise en compte de données relatives au trafic

Les données relatives au trafic sont disponibles auprès de cartographies accessibles, par exemple la base de données HERE précédemment citée. Les données à prendre en compte sont notamment la vitesse moyenne du trafic. Un indicateur de densité du trafic, usuellement désigné par l'acronyme JF (Jam Factor), peut être utilisé. Cet indicateur prend par exemple la valeur 0 lorsque le trafic est fluide et 1 en présence d'un trafic immobilisé. Cependant, les inventeurs considèrent que cet indicateur peut ne pas être nécessaire.

Etape 130 : prise en compte de données temporelles relatives à l'instant d'estimation

L'instant d'estimation peut être considéré comme appartenant à une journée, et à un segment temporel de la journée. Ainsi, l'instant d'estimation peut être associé à premier indicateur temporel, relatif au type de journée, ainsi qu'à un deuxième indicateur temporel, relatif au segment temporel de la journée auquel appartient l'instant d'estimation.

Le premier indicateur temporel peut être journée de travail / journée de week-end. Les inventeurs ont considéré qu'un indicateur optimal était :

- journée de travail, c'est-à-dire choisie parmi lundi, mardi, mercredi, jeudi, vendredi ;
- samedi ;
- dimanche.

Le deuxième indicateur temporel correspond à un segment temporel auquel appartient l'instant d'estimation au cours de la journée. Chaque journée est alors divisée en plusieurs segments temporels. Selon une première possibilité, les segments temporels sont : nuit ; matinée / après-midi / soirée, ce qui correspond respectivement aux plages horaires suivantes : 21h - 05h, 05h - 12h ; 12h - 17h ; 17h - 21h.

Selon une autre possibilité, préférée par les inventeurs, chaque journée est divisée en 24 segments temporels d'1 heure.

Etape 140 : prise en compte d'une pollution de fond.

Ce type de données d'entrée est optionnel. Il s'agit de prendre en compte un niveau "de fond", considéré comme uniforme sur plusieurs points de maillage, voire sur l'ensemble des points de maillage considérés, relatif à une concentration de l'analyte. Il s'agit d'une concentration provenant de sources de pollution autres que le trafic de véhicules. Il peut par exemple s'agir d'une pollution industrielle ou générée par le chauffage urbain. Ces données sont disponibles auprès de stations de référence disposées dans ou autour de l'environnement analysé. Lorsque plusieurs mesures sont disponibles, en différentes positions, une interpolation peut être effectuée au niveau de chaque point de maillage.

Etape 150 : estimation de la concentration d'au moins un premier analyte.

Au cours de cette étape, on utilise un premier réseau de neurones, de façon à estimer une concentration en au moins un premier analyte. Comme préalablement mentionné, le procédé peut être tel qu'un réseau de neurones, tel que décrit en lien avec la figure 2, est mis en oeuvre pour un seul analyte. A chaque analyte correspond un premier réseau de neurones spécifique. L'analyte peut par exemple être NOz ou PM10.

Pour ce type d'application, les inventeurs ont considéré que :

- la première couche cachée peut comporter un nombre de noeuds compris entre 200 et 1500 noeuds, par exemple égal à 500 noeuds pour NOz et 950 noeuds pour PM10;
- la deuxième couche cachée peut comporter un nombre de noeuds compris entre 2500 et 4500 noeuds, par exemple égal à 3350 noeuds pour $NO_2$ et 3800 noeuds pour PM10;

Le premier réseau de neurones peut également permettre une estimation simultanée de la concentration de plusieurs premiers analytes, par exemple $NO_2$ et PM10. Dans ce cas, le nombre de noeuds par couche est similaire à ceux précédemment indiqués. Une difficulté liée à ce mode de réalisation est que le nombre de stations de références mesurant simultanément ces deux analytes est réduit, ce qui limite le nombre de stations de références que l'on peut utiliser pour effectuer l'apprentissage du modèle.

Etape 160 estimation de la concentration d'au moins un deuxième analyte.

Au cours de cette étape, on met en oeuvre un deuxième réseau de neurones, adressant un deuxième analyte, considéré comme corrélé à un premier analyte dont une concentration a été estimée au cours de l'étape 150. Comme précédemment mentionné, il peut s'agir de $O_3$ (dépendant de $NO_2$ et de l'ensoleillement) ou de PM2.5 (corrélé à PM10).

Selon une possibilité, au cours de l'étape 150, on estime PM2.5 en tant que premier analyte et au cours de l'étape 160, on estime PM10 en tant que deuxième analyte.

Le deuxième réseau de neurones a une conception similaire au premier réseau de neurones. Cf. figure 2. Les données d'entrée peuvent notamment être :

- les données météorologiques, telles que définies dans l'étape 110 ;
- le type de journée et le segment temporel auquel appartient l'instant d'estimation (cf. étape 130) : les inventeurs ont constaté que le prise en compte de ces données, pourtant déjà considérées lors de l'étape 150, améliore la précision de l'estimation.
- au moins une concentration d'un premier analyte, préalablement estimée au cours de l'étape 150, le premier analyte étant considéré comme corrélé avec le deuxième analyte. Il peut s'agir d'une donnée différentielle, c'est-à-dire une variation de la concentration du premier analyte entre l'instant d'estimation et un instant d'estimation précédent.
- au moins un niveau de pollution de fond du premier analyte corrélé avec le deuxième analyte (cf. étape 140).

Les données d'entrée correspondant aux étapes 100 (configuration de la rue) et 120 (trafic) peuvent être prises en compte mais les inventeurs considèrent que cela n'est pas nécessaire, en particulier pour l'estimation de $O_3$ ou de PM2.5.

Selon un mode de réalisation, l'étape 160 est mise en oeuvre pour plusieurs deuxièmes analytes, par exemple $O_3$ et PM2.5. Dans ce cas, l'étape 160 utilise :

- soit deux deuxièmes réseaux de neurones distincts l'un de l'autre, chaque réseau de neurone étant configuré pour estimer la concentration d'un deuxième analyte. Dans cet exemple :

  • un deuxième réseau de neurones permet l'estimation de la concentration de $O_3$, en utilisant la concentration en NOz en tant que donnée d'entrée ;
  • et un deuxième réseau de neurones permet l'estimation de la concentration de PM2.5, en utilisant la concentration en PM10 comme une des données d'entrée.

- soit un même deuxième réseau de neurones, permettant une estimation simultanée de la concentration en différents deuxièmes analytes. Dans cet exemple, le deuxième réseau de neurones peut estimer la concentration de $O_3$ et la concentration de PM2.5 en utilisant les concentrations en $NO_2$ en PM10 en tant que donnée d'entrée.

La première approche, décrite dans le paragraphe précédent (un réseau de neurones différent pour chaque analyte) a été testée par les inventeurs et s'est avérée fonctionnelle.

Le recours à un deuxième réseau de neurones pour estimer chaque deuxième analyte permet d'adresser des analytes peu fréquemment mesurés par les stations de référence de type "trafic". En effet, pour ce type d'analyte, l'estimation d'une concentration par le biais d'un premier réseau de neurones, décrit en lien avec l'étape 150, est plus délicate, en raison du manque de données disponibles pour effectuer l'apprentissage. C'est par exemple le cas de $O_3$ ainsi que, en Europe, PM10. Les inventeurs ont estimés qu'il était plus pertinent d'utiliser la corrélation entre la concentration de chaque deuxième analyte avec la concentration d'au moins un premier analyte. Chaque deuxième analyte est alors estimé en utilisant un deuxième réseau de neurones dont une des données d'entrée est un premier analyte auquel le deuxième analyte est considéré comme corrélé.

Précisons que l'étape 160 est optionnelle. Le procédé peut se limiter à l'utilisation d'un premier réseau de neurones pour différents premiers analytes.

Etape 170 : réitération des étapes 100 à 160 à un autre instant d'estimation.

Essai expérimental

Les inventeurs ont effectué un apprentissage :

- de premiers réseaux de neurones, adressant respectivement NOz, PM10 ainsi que NOz et PM10;
- de deuxièmes réseaux de neurones, adressant respectivement $O_3$ ou PM2.5.

L'apprentissage a été effectué en utilisant des stations de référence basées en région parisienne, à proximité de rues dont on disposait de données quant au trafic. Suite à la phase d'apprentissage, des estimations ont été effectuées dans la ville de Grenoble, en différents instants d'estimation. Les concentrations estimées ont été confrontées à des valeurs de référence, mesurées par une station de référence. Elles ont également été confrontées à des estimations effectuées par le modèle analytique OSPM, décrit dans l'art antérieur.

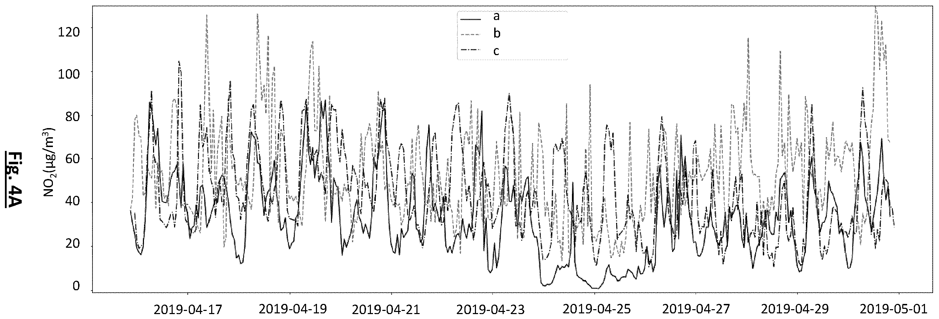

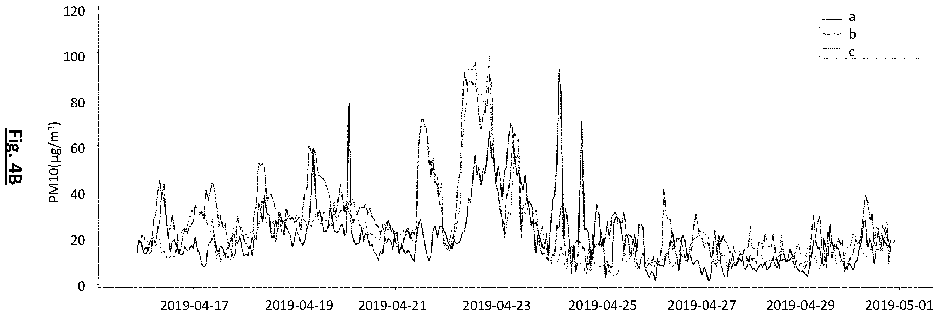

Au cours de ces essais, on a utilisé un premier réseau de neurones pour estimer la concentration en $NO_2$ et PM10. Les figures 4A et 4B montrent respectivement les concentrations estimées par le procédé selon l'invention (courbe c - traits mixtes), par OSPM (courbes b - tirets) et les mesures de référence (courbes a - traits pleins), en fonction du temps, respectivement pour $NO_2$ et PM10. Sur les figures 4A et 4B, l'axe des ordonnées correspond aux concentrations ($\mu g/m^3$) tandis que l'axe des abscisses correspond au décours temporel, sous la forme de dates au format année-mois-jour.

Sur la base des mesures réalisées, on a établi des indicateurs de performance de chaque modèle, en comparant les estimations avec les valeurs mesurées par la station de référence. Les indicateurs considérés sont l'erreur absolue moyenne (MAE), l'erreur quadratique moyenne (MSE) ainsi que le coefficient de corrélation R.

Les tableaux 1 et 2 montrent les indicateurs correspondant aux estimations représentées sur les figures 4A et 4B.

Tableau 1

| Modèle | MAE | MSE | R |
|---|---|---|---|
| Invention | 18.60 | 24.00 | 0.42 |
| OSPM | 26.53 | 32.23 | 0.28 |

Tableau 2

| Modèle | MAE | MSE | R |
|---|---|---|---|
| Invention | 11.46 | 16.02 | 0.56 |
| OSPM | 10.89 | 16.29 | 0.46 |

Pour chaque analyte, on constate que l'invention permet d'obtenir des meilleurs indicateurs : les indicateurs MAE et MSE sont plus faibles, notamment pour $NO_2$, et la corrélation est plus élevée.

Sur la base des concentrations de NO2 et PM10, on a également respectivement estimé les concentrations de $O_3$ et PM2.5.

Des essais de validation ont été effectués à proximité de stations de référence permettant d'accéder à des concentrations mesurées de $O_3$ (Station 1 et Station 2) et PM 2.5 (Station 3 et Station 4).

Les tableaux 3 et 4 montrent, pour deux stations de référence, l'erreur absolue moyenne (MAE), l'erreur quadratique moyenne (MSE) ainsi que le coefficient de corrélation R, issus de la comparaison entre l'estimation et les mesures données par la station de référence, respectivement pour $O_3$ et PM2.5.

Tableau 3

| Station de reference | MAE | MSE | R |
|---|---|---|---|
| Station 1 | 12.71 | 15.53 | 0.82 |
| Station 2 | 13.01 | 15.86 | 0.86 |

Tableau 4

| Station de reference | MAE | MSE | R |
|---|---|---|---|
| Station 3 | 2.31 | 2.99 | 0.57 |
| Station 4 | 3.01 | 4.16 | 0.84 |

Un avantage de l'invention est que la plupart des données d'entrée sont disponibles dans des bases de données spécialisées, aisément accessibles, et fréquemment remises à jour. Ainsi, les estimations des concentrations sont réalisées en fonction des conditions que l'on peut considérer comme en temps réel. Par ailleurs, l'invention permet de mettre en oeuvre l'invention selon un maillage fin, indépendamment de la position ou de la densité des stations de référence. L'invention permet l'obtention de cartographies de haute résolution spatiale, régulièrement mises à jour.

L'invention pourra être mise en oeuvre pour estimer des concentrations d'analytes, en particulier dans des environnements comportant des voies parcourues par des véhicules. La principale application visée est la réalisation de cartographies en milieu urbain ou périurbain, mais d'autres applications sont envisageables, par exemple la réalisation de cartographies au voisinage d'autoroutes, ou de centre commerciaux ou d'installations aéroportuaires.

**Revendications**

**1.** Procédé d'estimation d'une concentration d'analytes dans un environnement urbain ou péri-urbain comportant des voies configurées pour être traversées par un trafic de véhicules, le procédé comportant les étapes suivantes :

a) maillage spatial de l'environnement, de façon à obtenir des points de maillage ;
b) prise en compte, au niveau de chaque point de maillage, à un instant d'estimation :

- d'au moins une donnée fixe dans le temps, relative à chaque voie ;
- de données dynamiques, estimées ou mesurées, variables en fonction de l'instant d'estimation, comportant des données météorologiques, des données relatives au trafic, et des données temporelles relatives à l'instant d'estimation ;

c) utilisation des données prises en compte lors de l'étape b), à l'instant d'estimation, en tant que données d'entrée d'un premier algorithme d'intelligence artificielle supervisé, de façon à estimer, au niveau de chaque point de maillage, une concentration d'au moins un premier analyte, à l'instant d'estimation ;
d) utilisation de la concentration du premier analyte, résultant de l'étape c), à au moins un point de maillage à l'instant d'estimation, en tant que donnée d'entrée d'un deuxième algorithme d'intelligence artificielle supervisé, différent du premier algorithme d'intelligence artificielle supervisé, de façon à estimer, au point de maillage une concentration d'au moins un deuxième analyte, différent du premier analyte, à l'instant d'estimation ;

les étapes a) à d) étant mises en oeuvre par une unité de traitement, comportant un microprocesseur.

**2.** Procédé selon la revendication 1, dans lequel la concentration du deuxième analyte est considérée comme corrélée avec la concentration du premier analyte.

**3.** Procédé selon l'une quelconque des revendications précédentes, dans lequel lors de l'étape b), les données météorologiques comportent :

- la direction du vent ;

- et/ou la vitesse du vent.

4. Procédé selon l'une quelconque des revendications précédentes, dans lequel les données météorologiques comportent également au moins une des données suivantes : température ; pression ; taux d'humidité ; nébulosité.

5. Procédé selon l'une quelconque des revendications précédentes, dans lequel les données relatives au trafic routier comportent au moins une vitesse moyenne des véhicules et/ou un niveau d'intensité du trafic.

6. Procédé selon l'une quelconque des revendications précédentes, dans lequel les données temporelles correspondant à l'instant d'estimation comportent au moins une des données suivantes :

   - type de journée parmi des types de journées prédéterminés à laquelle appartient l'instant d'estimation ;
   - segment temporel auquel appartient l'instant d'estimation, chaque journée étant divisée en segments temporels.

7. Procédé selon l'une quelconque des revendications précédentes, dans lequel le premier algorithme est un premier réseau de neurones, comportant :

   - une couche d'entrée, comportant les données d'entrée prises en compte lors de l'étape b) ;
   - au moins une couche cachée, et de préférence au moins deux couches cachées, comportant des noeuds ;
   - une couche de sortie, comportant une estimation d'une concentration du premier analyte.

8. Procédé selon la revendication 7, dans lequel le premier réseau de neurones comporte deux couches cachées.

9. Procédé selon la revendication 8, dans lequel :

   - la première couche cachée du premier réseau de neurones comporte entre 200 et 800 noeuds ;
   - la deuxième couche cachée du premier réseau de neurones comporte entre 2500 et 4500 noeuds.

10. Procédé selon l'une quelconque des revendications précédentes, dans lequel dans lequel le deuxième algorithme est un deuxième réseau de neurones comportant :

    - une couche d'entrée, comportant des données d'entrée prises en compte lors de l'étape b), ainsi qu'une concentration d'un premier analyte, estimée à l'instant d'estimation, au cours de l'étape c);
    - au moins une couche cachée, et de préférence au moins deux couches cachées, comportant des noeuds ;
    - une couche de sortie, comportant une estimation d'une concentration du deuxième analyte, à l'instant d'estimation.

11. Procédé selon la revendication 10, dans lequel le deuxième réseau de neurones comporte deux couches.

12. Procédé selon la revendication 11, dans lequel :

    - la première couche cachée du deuxième réseau de neurones comporte entre 1000 et 2000 noeuds ;
    - la deuxième couche cachée du deuxième réseau de neurones comporte entre 2500 et 4500 noeuds.

13. Procédé selon l'une quelconque des revendications 10 à 12, dans lequel l'étape d) comporte une estimation de la concentration d'au moins deux deuxièmes analytes différents, en utilisant respectivement au moins deux deuxièmes réseaux de neurones différents, chaque deuxième réseau de neurones permettant une estimation d'une concentration d'un deuxième analyte.


**Patentansprüche**

1. Verfahren zum Schätzen einer Konzentration von Analyten in einer städtischen oder stadtnahen Umgebung, die Wege umfasst, die dazu ausgestaltet sind, von einem Fahrzeugverkehr überquert zu werden, wobei das Verfahren die folgenden Schritte umfasst:

   a) räumliches Vermaschen der Umgebung, so dass Vermaschungspunkte erhalten werden;

b) Berücksichtigen, an jedem Vermaschungspunkt, zu einem Schätzzeitpunkt:

- mindestens eines zeitlich feststehenden Datenelements bezüglich jedes Wegs;
- von geschätzten oder gemessenen dynamischen Daten, die in Abhängigkeit vom Schätzzeitpunkt variabel sind, umfassend meteorologische Daten, verkehrsbezogene Daten und schätzzeitpunktbezogene zeitliche Daten;

c) Verwenden der beim Schritt b) zu dem Schätzzeitpunkt berücksichtigten Daten als Eingangsdaten eines ersten überwachten Algorithmus der künstlichen Intelligenz, so dass, an jedem Vermaschungspunkt, eine Konzentration mindestens eines ersten Analyten zu dem Schätzzeitpunkt geschätzt wird;

d) Verwenden der Konzentration des ersten Analyten, die aus dem Schritt c) resultiert, an mindestens einem Vermaschungspunkt zu dem Schätzzeitpunkt als Eingangsdaten eines zweiten überwachten Algorithmus der künstlichen Intelligenz, der verschieden von dem ersten überwachten Algorithmus der künstlichen Intelligenz ist, so dass an dem Vermaschungspunkt eine Konzentration mindestens eines zweiten Analyten, der von dem ersten Analyten verschieden ist, zu dem Schätzzeitpunkt geschätzt wird;

wobei die Schritte a) bis d) von einer Verarbeitungseinheit umgesetzt werden, die einen Mikroprozessor umfasst.

2. Verfahren nach Anspruch 1, bei dem die Konzentration des zweiten Analyten als mit der Konzentration des ersten Analyten korreliert betrachtet wird.

3. Verfahren nach einem der vorhergehenden Ansprüche, bei dem beim Schritt b) die meteorologischen Daten umfassen:

- die Windrichtung;
- und/oder die Windgeschwindigkeit.

4. Verfahren nach einem der vorhergehenden Ansprüche, bei dem die meteorologischen Daten auch mindestens eines der folgenden Datenelemente umfassen: Temperatur; Druck; Feuchtigkeitsgehalt; Bewölkung.

5. Verfahren nach einem der vorhergehenden Ansprüche, bei dem die straßenverkehrsbezogenen Daten mindestens eine Durchschnittsgeschwindigkeit der Fahrzeuge und/oder ein Intensitätsniveau des Verkehrs umfassen.

6. Verfahren nach einem der vorhergehenden Ansprüche, bei dem die dem Schätzzeitpunkt entsprechenden zeitlichen Daten mindestens eines der folgenden Datenelemente umfassen:

- Tagestyp unter vorbestimmten Tagestypen, dem der Schätzzeitpunkt angehört;
- Zeitsegment, dem der Schätzzeitpunkt angehört, wobei jeder Tag in Zeitsegmente unterteilt ist.

7. Verfahren nach einem der vorhergehenden Ansprüche, bei dem der erste Algorithmus ein erstes neuronales Netz ist, umfassend:

- eine Eingabeschicht, welche die beim Schritt b) berücksichtigten Eingangsdaten umfasst;
- mindestens eine verborgene Schicht und bevorzugt mindestens zwei verborgene Schichten, Knoten umfassend;
- eine Ausgabeschicht, umfassend eine Schätzung einer Konzentration des ersten Analyten.

8. Verfahren nach Anspruch 7, bei dem das erste neuronale Netz zwei verborgene Schichten umfasst.

9. Verfahren nach Anspruch 8, bei dem:

- die erste verborgene Schicht des ersten neuronalen Netzes zwischen 200 und 800 Knoten umfasst;
- die zweite verborgene Schicht des ersten neuronalen Netzes zwischen 2500 und 4500 Knoten umfasst.

10. Verfahren nach einem der vorhergehenden Ansprüche, bei dem der zweite Algorithmus ein zweites neuronales Netz ist, umfassend:

- eine Eingabeschicht, umfassend beim Schritt b) berücksichtigte Eingangsdaten sowie eine Konzentration eines ersten Analyten, die zum Schätzzeitpunkt während des Schritts c) geschätzt wurde;
- mindestens eine verborgene Schicht und bevorzugt mindestens zwei verborgene Schichten, Knoten umfassend;
- eine Ausgabeschicht, umfassend eine Schätzung einer Konzentration des zweiten Analysten zu dem Schätzzeitpunkt.

11. Verfahren nach Anspruch 10, bei dem das zweite neuronale Netz zwei Schichten umfasst.

12. Verfahren nach Anspruch 11, bei dem:

- die erste verborgene Schicht des zweiten neuronalen Netzes zwischen 1000 und 2000 Knoten umfasst;
- die zweite verborgene Schicht des zweiten neuronalen Netzes zwischen 2500 und 4500 Knoten umfasst.

13. Verfahren nach einem der Ansprüche 10 bis 12, bei dem der Schritt d) ein Schätzen der Konzentration von mindestens zwei verschiedenen zweiten Analyten umfasst, wobei jeweils mindestens zwei verschiedene zweite neuronale Netze verwendet werden, wobei jedes zweite neuronale Netz ein Schätzen einer Konzentration eines zweiten Analyten ermöglicht.

**Claims**

1. Method for estimating an analyte concentration in an urban or peri-urban environment containing lanes configured to be followed by vehicular traffic, the method comprising the following steps:

   a) generating a spatial mesh of the environment, so as to obtain mesh points;
   b) taking into account, at each mesh point, at an estimation time:

      - at least one datum that remains constant over time, relating to each lane;
      - estimated or measured dynamic data that vary as a function of the estimation time, these data including meteorological data, traffic data, and time-dependent data relating to the estimation time;

   c) using the data taken into account in step b), at the estimation time, as input data for a first supervised artificial-intelligence algorithm, so as to estimate, at each mesh point, a concentration of at least a first analyte, at the estimation time;
   d) using the concentration of the first analyte, resulting from step c), at at least one mesh point at the estimation time, as input datum for a second supervised artificial-intelligence algorithm that is different from the first supervised artificial-intelligence algorithm, so as to estimate, at the mesh point, a concentration of at least a second analyte that is different from the first analyte, at the estimation time;

   steps a) to d) being implemented by a processing unit, comprising a microprocessor.

2. Method according to Claim 1, wherein the concentration of the second analyte is considered to be correlated with the concentration of the first analyte.

3. Method according to any one of the preceding claims, wherein, in step b), the meteorological data comprise:

   - wind direction;
   - and/or wind speed.

4. Method according to any one of the preceding claims, wherein the meteorological data also comprise at least one of the following data: temperature; pressure; humidity level; cloudiness.

5. Method according to any one of the preceding claims, wherein the traffic data comprise at least an average vehicle speed and/or a traffic intensity level.

6. Method according to any one of the preceding claims, wherein the time-dependent data corresponding to the estimation time comprise at least one of the following data:

- type of day among predetermined types of days to which the estimation time belongs;
- time segment to which the estimation time belongs, each day being divided into time segments.

7. Method according to any one of the preceding claims, wherein the first algorithm is a first neural network, comprising:

   - an input layer, comprising the input data taken into account in step b);
   - at least one hidden layer, and preferably at least two hidden layers, comprising nodes;
   - an output layer, comprising an estimation of a concentration of the first analyte.

8. Method according to Claim 7, wherein the first neural network comprises two hidden layers.

9. Method according to Claim 8, wherein:

   - the first hidden layer of the first neural network comprises between 200 and 800 nodes;
   - the second hidden layer of the first neural network comprises between 2500 and 4500 nodes.

10. Method according to any one of the preceding claims, wherein the second algorithm is a second neural network comprising:

   - an input layer, comprising input data taken into account in step b), and a concentration of a first analyte estimated at the estimation time in step c);
   - at least one hidden layer, and preferably at least two hidden layers, comprising nodes;
   - an output layer, comprising an estimation of a concentration of the second analyte, at the estimation time.

11. Method according to Claim 10, wherein the second neural network comprises two layers.

12. Method according to Claim 11, wherein:

   - the first hidden layer of the second neural network comprises between 1000 and 2000 nodes;
   - the second hidden layer of the second neural network comprises between 2500 and 4500 nodes.

13. Method according to any one of Claims 10 to 12, wherein step d) comprises estimating the concentration of at least two different second analytes, using at least two different second neural networks, respectively, each second neural network allowing a concentration of a second analyte to be estimated.

**Fig. 1**

**Fig. 2**

**Fig. 3**

**Fig. 4A**

**Fig. 4B**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- CN 106056210

**Littérature non-brevet citée dans la description**

- **A. SULEIMAN et al.** Applying machine learning methods in managing urban concentrations of traffic-related particulate matter (PM10 and PM2.5). *ATMOSPHERIC POLLUTION RESEARCH,* 06 Juillet 2018, vol. 10 (1), ISSN 1309-1042, 134-144